# EUROPEAN PATENT APPLICATION

(11) **EP 1 994 935 A1**
(43) Date of publication of application: **26.11.2008**
(21) Application number: 08156245.6
(22) Date of filing: 15.05.2008
(51) Int. Cl.: A61K 33/00

(54) **Use of xenon to protect organs against ischaemic damage**

(30) Priority: 22.05.2007 IT MI20071031
(71) Applicant: Societa' Italiana Acetilene & Derivati S.I.A.D. S.p.A. in abbreviated form SIAD S.p.A., 24126 Bergamo (IT)
(72) Inventor: Giunta, Francesco, 24126, BERGAMO (IT); Bissolotti, Giorgio, 24126, BERGAMO (IT)
(74) Representative: Minoja, Fabrizio

(57) **Abstract**

Disclosed is the use of xenon blended with oxygen for the preparation of a medicament for acute administration to protect the organs against damage resulting from trauma.

## Description

The present invention relates to the use of xenon blended with oxygen for the preparation of a medicament for acute administration to protect the organs against damage resulting from trauma.

State of the art

Xenon has been used experimentally in clinical anaesthesia for over 50 years [a]. It is a gas of proven efficacy and clinical atoxicity, and possesses a very favourable environmental safety profile [b], with just one drawback: its cost [c].

Numerous clinical trials, especially in the last decade, have almost unanimously demonstrated four basic characteristics possessed by xenon:
1) A highly favourable pharmacokinetic profile with a very low blood/gas partition coefficient, and consequently an extremely rapid onset and offset [d, e and f];
2) Profound analgesia [g, h];
3) A favourable cardiovascular profile and no adverse effects on inotropism or vascular tone [i, j, k, and 1];
4) Organ protection: protection against ischaemic, hypoxic and excitotoxic brain damage; myocardial preconditioning [m, n, o and p].

Studies of xenon have demonstrated that these effects are developed through phenomena which are complex, and not yet fully understood. A range of these phenomena is illustrated in the studies reported in the references, relating to interference by xenon with neurotransmitters.

Glutamate is the most important excitatory neurotransmitter in the central nervous system of mammals, and essential to its correct operation. The excitatory amino acid glutamate (Glu) can increase neurotransmission and cause long-term strengthening of the central synapses. Exogenous Glu and its agonists which mimic the effects of the endogenous transmitter can act on the Glu receptors, with reuptake by Glu transporters, thus altering the normal neurophysiological functions. High extracellular levels of Glu agonists can induce a flow of calcium and sodium ions into the cells, especially through the bond to the NMDA receptors. The presence of excessive amounts of glutamate in the synaptic cleft can lead to neurone death.

The term "excitotoxicity", coined by Olney [q], denotes the process whereby the hyperactivation of glutamate receptors, especially those of subtype NMDA (N-methyl-D-aspartate), leads to an intracellular calcium overload and triggers a biochemical cascade that causes the death of neurones exposed to high concentrations of the neurotransmitter. The same pathological process is believed to be responsible for maintenance of acute neuronal damage (stroke, head injury, etc.), but is also the pathological event responsible for the slow neuronal deterioration that takes place in chronic neurodegenerative diseases (SLA, Parkinson's disease, Alzheimer's disease, etc.) [p].

Many studies have consequently been conducted to investigate how a molecule with NMDA-antagonist properties acts towards excitotoxic damage, and to evaluate its possible clinical use as a "neuroprotective medicament".

Ketamine, like phencyclidine (PCP) and dizocilpine (MK101), are potent NMDA receptor inhibitors, but their clinical use is prevented by the profound psychological and behavioural changes they can cause. These molecules also cause major histological degenerative modifications of the posterior cingulate gyrus and the retrosplenial cortex, which may be responsible for their psychotomimetic effect [r, s, t]. Nitrogen protoxide possesses similar characteristics because it also has NMDA-blocking properties [u], but it has some side effects which restrict its use. Many experimental findings indicate that NMDA receptor antagonists can be neuroprotective in neuronal damage models in vitro and in vivo (Sarraf-Yazdi et al., 1998; Harada et al., 1999; Popovic et al., 2000; Kudo et al., 2001), and can also prevent deterioration of the cognitive function after a cardiopulmonary bypass (Arrowsmith et al., 1998; Franks et al., 1998; de Sousa et al., 2000; Yamakura e Harris, 2000; Nagata et al., 2001; Ma et al., 2002; Wilhelm et al., 2002; Ma et al., 2003). Despite the hypothesis of a neuroprotective effect in animal models, many NMDA receptor antagonists have demonstrated intrinsic neurotoxicity (characterised by a specific lesion of the retrosplenial cortex) or an unfavourable pharmacokinetic profile (Arrowsmith et al., 1998).

Although it remains to be demonstrated whether these effects are determined merely by simple NMDA antagonism, or disturbance of other neurotransmission systems is also required, it is certain that xenon, which is a non-competitive NMDA receptor inhibitor [v, w], presents none of these drawbacks. Xenon also seems able to reduce the release of dopamine [x], a neurotransmitter directly involved in inducing and maintaining psychotic states; the activity of the dopaminergic system seems to be boosted by all other molecules with NMDA-antagonist activity [y, z, aa]. Recent studies have demonstrated that xenon, the only volatile anaesthetic to possess NMDA receptor antagonist properties, does not induce neurotoxicity in the retrosplenial cortex (Ma et al., 2002), but protects it against the neurotoxic damage produced by ketamine (Nagata et al., 2001) and reduces the damage caused by excitatory aminoacids (Ma et al., 2002; Wilhelm et al., 2002). Moreover, xenon quickly crosses the blood-brain barrier (BBB), thus rapidly reaching the site of the damage. It was recently demonstrated that xenon is an NMDA receptor antagonist (Franks et al., 1998; de Sousa et al., 2000; Yamakura and Harris, 2000).

Earlier studies which experimented with xenon ruled out ultrastructural cytotoxic effects (Natale et al., 2002) and macroscopic damage to the main organs (Natale et al., 1998; Burov et al., 1998), but the central nervous system has never been evaluated by electron microscopy.

Although there is now strong evidence from preclinical research in the literature about the possibility of using xenon as an organ protector, no protocol for out-patient clinical use has yet been introduced. No etiological treatment is currently available for ischaemic damage, and purely symptomatic treatment is routinely administered at the outset.

### Description of the invention

It has now been found that xenon blended with oxygen, if administered to trauma patients at an early stage, effectively prevents and counteracts ischaemic organ damage, especially to the heart and brain.

The activity of xenon blended with oxygen has been demonstrated by a morphological study whose purpose was the qualitative and quantitative evaluation of the effect of xenon on excitotoxic damage induced by an NMDA agonist in the CNS of the rat.

The invention therefore relates to the use of xenon blended with oxygen for the preparation of a medicament for acute administration to protect the organs against damage resulting from trauma.

The medicament is preferably administered within 20 minutes of the trauma, by inhalation or intravenously, to minimise the natural apoptotic activity.

The mixture has the following preferred composition:
- 40% - 80% vol of xenon;
- 60% - 20% vol of oxygen
and can also include other rare gases, especially krypton, so that the oxygen concentration exceeds 20% vol. The optional presence of other rare gases may have important effects as regards the purity of the xenon used to prepare the mixtures administered, because a blend of xenon with other rare gases, such as krypton, could be used as starting gas.

The medicament could advantageously be administered at the emergency stage: for this purpose, mobile emergency units (ambulances, air ambulances, etc.) could easily be fitted with equipment designed to deliver the gaseous xenon/oxygen blend for inhalatory administration. The xenon could also be administered intravenously, as described in IT1312017.

As already mentioned, the novel use according to the invention has been demonstrated on an animal model of acute excitotoxic damage induced by the administration of N-methyl-DL-aspartic acid (NMA) in the arcuate nucleus of the rat, and the morphological effects of the acute tissue damage have been investigated.

Tests have been carried out to establish the effects of xenon on the neurones of the arcuate nucleus in the rat from the morphological standpoint, whether the administration of xenon together with the toxin can reduce the damage, and whether the neuroprotection can be maintained over time.

The results demonstrate that NMA has a toxic effect on the neurones of the AN and that xenon is not only devoid of intrinsic toxicity, but can also prevent excitotoxic damage to the AN.

In the arcuate nucleus of the control rats, the neurones and glial cells corresponded to the standard descriptions.

In preparations made from the rats treated with NMA, staining with cresyl violet and haematoxylin-eosin showed a neuronal loss typically characterised by cell degeneration and identifiable by pycnotic nuclei and swelling of the cytoplasmic compartment which gives the typical "bull's-eye" appearance.

According to current knowledge, the distinction between cell apoptosis and cell necrosis is becoming more and more blurred. Degenerating neurones present a typical morphological profile of both apoptosis and necrosis. Development towards one of the two forms of cell death depends mainly on the specific environmental microconditions of each cell, and in theory on the dose-response relationship with the neurotoxin (in ischaemic damage, this phenomenon is known as "the maturation phenomenon").

In the animals treated with NMA, we observed different morphological images at the different experimental times when the animals were sacrificed (3 hours, 24 hours and 7 days), always attributable to the specific excitotoxic damage and the inflammatory astrocytic response secondary to neuronal death (gliosis).

In practice, this means that the damage after 3 hours is critical, and a reactive response by the glia can be observed after 24 hours; finally, the damage seems to stabilise after 7 days.

The data obtained by fluorescence microscopy (FJB) are interesting, and particularly significant.

The fluorescence appears markedly positive in the arcuate nuclei of the animals sacrificed after 3 hours. FJB staining only marks the degenerating neurones in which particular compounds are released into the cytoplasm. It is therefore possible that while the majority of the neurones die within a short time, the others may develop a degenerative process, but only after a few days, when signs of damage can be demonstrated by some traces of positivity to FJB fluorescence. All the animals treated with NMA and subsequently exposed to 70% xenon, studied at any time and with any method, presented a significant reduction in excitotoxic damage (Abramo et al, 2004).

The neurones of arcuate nuclei from the rats treated with xenon and observed under the optical microscope correspond to the descriptions obtained from the rats belonging to the control group and stained with haematoxylin-eosin and cresyl violet; they also appear negative to FJB, and the fluorescence of the nuclei stained with DAPI, which marks the viable cells, is intensely positive.

Equally, no morphological alteration induced by xenon was observed in the hippocampus, whose cells are considered particularly sensitive to injury such as hypoxia or ischaemia, and to neurotoxic damage.

We did not observe any behavioural alteration in the animals during the period of exposure to xenon: this finding confirms the good neuropsycholeptic profile of the gas.

Xenon acts in accordance with the data on the etiology and physiopathology of acute damage, and delays or otherwise interferes with the mechanisms underlying subacute damage. It can also be assumed that excitotoxic damage occurs with a latency time during which xenon seems to operate at both molecular and cell level (in the glia-neurone ratios).

From the qualitative and quantitative standpoints, the results demonstrate an important neuroprotective effect on the nerve tissue, and this action seems to involve the nerve cells at the early stage and the glia at a later stage.

These preliminary results clearly demonstrate the phenomenon of neuroprotection.

In conclusion, our results confirm that:
- NMA induces major neurone damage in the arcuate nucleus of the rat, which is already evident 3 hours after the administration of the substance. A marked reduction in the number of viable neurones can be observed under the optical microscope. However, cell loss is not only dependent on the dose of toxin, but also on time.
- Xenon has no neurotoxic properties from the standpoint of morphological evaluation. Xenon seems to attenuate excitotoxic damage from its onset and, although it was administered after NMA, its unique neuroprotective role was confirmed to prevent the cascade of events secondary to the damage.

### References

a) Cullen SC, Gross EG. The anaesthetic properties of xenon in animals and human beings, with additional observations on krypton. Science 1951; 113: 580-2.
b) Sanders RD, Franks NP, Maze M. Xenon: no stranger to anaesthesia. Br J Anaesth. 2003 Nov;91(5):709-17. Lachmann B, Armbruster S, Schairer W, et al. Safety and efficacy of xenon in routine use as an inhalational anaesthetic. Lancet 1990; 335: 141-5.
c) Nakata Y, Goto T, Niimi Y, Morita S. Cost analysis of xenon anesthesia: a comparison with nitrous oxide-isoflurane and nitrous oxide-sevoflurane anesthesia. J Clin Anesth 1999; 11:477-81.
d) (Goto T, Saito H, Shinkai M, Nakata Y, Ichinose F, Morita S. Xenon provides faster emergence from anesthesia than does nitrous oxide-sevoflurane or nitrous oxide-isoflurane. Anesthesiology 1997; 86: 1273-8.
e) Goto T, Suwa K, Uezono S, Ichinose F, Uchiyama M, Morita S. The blood-gas partition coefficient of xenon may be lower than generally accepted. Br J Anaesth 1998; 80: 255-6.
f) Nakata Y, Goto T, Morita S. Comparison of inhalation inductions with xenon and sevoflurane. Acta Anaesthesiol Scand 1997; 41: 1157-61).
g) Petersen-Felix S, Luginbuhl M, Schnider TW, Curatolo M, Arendt-Nielsen L, Zbinden AM. Comparison of the analgesic potency of xenon and nitrous oxide in humans evaluated by experimental pain. Br J Anaesth. 1998 Nov;81(5):742-7.
h) Yagi M, Mashimo T, Kawaguchi T, Yoshiya I. Analgesic and hypnotic effects of subanaesthetic concentrations of xenon in human volunteers: comparison with nitrous oxide. Br J Anaesth. 1995 Jun;74(6):670-3).
i) Boomsma F, Rupreht J, Veld AJ, de Jong FH, Dzoljic M, Lachmann B. Haemodynamic and neurohumoral effects of xenon anaesthesia. A comparison with nitrous oxide. Anaesthesia 1990; 45: 273.
j) Dingley J, King R, Hughes L, et al. Exploration of xenon as a potential cardiostable sedative: a comparison with propofol after cardiac surgery. Anaesthesia 2001; 56: 829-35.
k) Lachmann B, Armbruster S, Schairer W, et al. Safety and efficacy of xenon in routine use as an inhalational anaesthetic. Lancet 1990; 335: 1413-5.
l) Luttropp HH, Romner B, Perhag L, Eskilsson J, Fredriksen S, Werner O. Left ventricular performance and cerebral haemodynamics during xenon anaesthesia. A transoesophageal echocardiography and transcranial Doppler sonography study. Anaesthesia 1993; 48: 1045-9.
m) Wilhelm S, Ma D, Maze M, Franks NP. Effects of xenon on in vitro and in vivo models of neuronal injury. Anesthesiology 2002; 96: 1485-91.
n) Petzelt C, Blom P, Schmehl W, Muller J, Kox WJ. Prevention of neurotoxicity in hypoxic cortical neurons by the noble gas xenon. Life Sci. 2003 Mar 14;72(17):1909-18.
o) Weber NC, Toma O, Wolter JI, Obal D, Mullenheim J, Preckel B, Schlack W. The noble gas xenon induces pharmacological preconditioning in the rat heart in vivo via induction of PKC-epsilon and p38 MAPK. Br J Pharmacol. 2005 Jan;144(1):123-32.
p) Preckel B, Weber NC, Sanders RD, Maze M, Schlack W. Molecular mechanisms transducing the anesthetic, analgesic, and organ-protective actions of xenon. Anesthesiology. 2006 Jul; 105(1): 187-97).
q) Olney JW. Brain lesions, obesity, and other disturbances in mice treated with monosodium glutamate. Science 1969; 164: 719-21.
r) Choi DW, Koh JY, Peters S. Pharmacology of glutamate neurotoxicity in cortical cell culture: attenuation by NMDA antagonists. J Neurosci 1988; 8: 185-96.
s) Malhotra AK, Pinals DA, Weingartner H, et al. NMDA receptor function and human cognition: the effects of ketamine in healthy volunteers. Neuropsychopharmacology 1996; 14: 301-7.
t) Olney JW, Labruyere J, Price MT. Pathological changes induced in cerebrocortical neurones by phencyclidine and related drugs. Science 1989; 244: 1360-2.
u) Allen HL, Iversen LL. Phencyclidine, dizocilpine, and cerebrocortical neurones. Science 1990; 247: 221.
v) Jevtovic-Todorovic V, Todorovic SM, Mennerick S, et al. Nitrous oxide (laughing gas) is an NMDA antagonist, neuroprotectant and neurotoxin. Nature Med 1998; 4: 460-3.
w) Franks NP, Dickinson R, de Sousa SLM, Hall AC, Lieb WR. How does xenon produce anaesthesia? Nature 1998; 396: 324.
x) De Sousa SL, Dickinson R, Lieb WR, Franks NP. Contrasting synaptic actions of the inhalational general anesthetics isoflurane and xenon. Anesthesiology 2000; 92: 1055-66.
y) Petzelt CH. New concepts in neuroprotection. J Anasth Intensivbehandlung 2001; 3: S38.
z) Murakawa M, Adachi T, Nakao S, Seo N, Shingu K, Mori K. Activation of the cortical and medullary dopaminergic systems by nitrous oxide in rats: a possible neurochemical basis for psychotropic effects and postanesthetic nausea and vomiting. Anesth Analg 1994; 78: 376-81.

aa) Moghaddam B, Adams B, Verma A, Daly D. Activation of glutamatergic neurotransmission by ketamine: a novel step in the pathway from NMDA receptor blockade to dopaminergic and cognitive disruptions associated with the prefrontal cortex. J Neurosci 1997; 17: 2921-7.
ab) Lindefors N, Barati S, O'Connor WT. Differential effects of single and repeated ketamine administration on dopamine, serotonin and GABA transmission in rat medial prefrontal cortex. Brain Res 1997; 759: 205-12.

## Claims

1. Use of xenon blended with oxygen for the preparation of a medicament for acute administration to protect the organs against damage resulting from trauma.

2. Use as claimed in claim 1, wherein the medicament is preferably administered within 20 minutes of the trauma.

3. Use as claimed in claim 1 or 2, wherein the medicament is administered by inhalation or intravenously.

4. Use as claimed in any of claims 1-3, wherein the medicament protects the organs against ischaemic heart, kidney or brain damage.

5. Use as claimed in claim 1 by inhalation, wherein the mixture has the following composition:
- 40% - 80% vol of xenon;
- 60% - 20% vol of oxygen.

6. Use as claimed in any of claims 1 to 5, wherein the mixture includes other rare gases, in particular krypton, at any concentration, so that the oxygen concentration exceeds 20%.
